# EUROPÄISCHE PATENTSCHRIFT

(11) **EP 0 396 077 B1**
(45) Veröffentlichungstag und Bekanntmachung des Hinweises auf die Patenterteilung: **07.04.1993**
(21) Anmeldenummer: 90108211.5
(22) Anmeldetag: 30.04.1990
(51) Int. Cl.: C07C 309/46, C07C 309/51, C07C 303/22

(54) **Neue Phenylendiamine sowie ein Verfahren zur Herstellung von Phenylendiaminen**
Phenylene diamines and a process for their preparation
Phénylène diamines et un procédé pour leur préparation

(30) Priorität: 03.05.1989 DE 3914650
(43) Veröffentlichungstag der Anmeldung: 07.11.1990
(73) Patentinhaber: BASF Aktiengesellschaft, 67063 Ludwigshafen (DE)
(72) Erfinder: Pandl, Klaus, Dr., D-6700 Ludwigshafen (DE); Patsch, Manfred, Dr., D-6706 Wachenheim (DE)

(56) Entgegenhaltungen:
- EP-A- 0 038 296
- EP-A- 0 315 045
- GB-A- 333 559
- US-A- 3 232 980
- US-A- 3 444 156

## Beschreibung

Die vorliegende Erfindung betrifft Phenylendiamine der Formel I 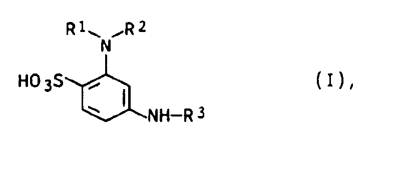
in der
R¹ Wasserstoff, Formyl oder Oxalyl,
R² Wasserstoff oder C₁-C₄-Alkyl und
R³ Wasserstoff oder C₂-C₈-Alkanoyl bedeuten,
mit der Maßgabe, daß R¹ und R² nicht gleichzeitig für Wasserstoff stehen und daß, wenn R² Methyl oder Ethyl bedeutet, R¹ und R³ nicht gleichzeitig für Wasserstoff stehen, sowie ein Verfahren zur Herstellung von Phenylendiaminen.

Aus der älteren Patentanmeldung EP-A-315 045 ist 4-Amino-2-(N-methylamino)benzolsulfonsäure sowie 4-Amino-2-(N-ethylamino)benzolsulfonsäure bekannt.

Aufgabe der vorliegenden Erfindung war es, neue Phenylendiamine sowie ein Verfahren zu ihrer Herstellung bereitzustellen.

Demgemäß wurden die eingangs näher bezeichneten Phenylendiamine der Formel I gefunden.

Alle in der obengenannten Formel I auftretenden Alkylgruppen können sowohl geradkettig als auch verzweigt sein.

R² steht beispielsweise für Methyl, Ethyl, Propyl, Isopropyl, Butyl, Isobutyl oder sec-Butyl.

R³ steht beispielsweise für Acetyl, Propionyl, Butyryl, Isobutyryl, Pentanoyl, Hexanoyl, Heptanoyl, Octanoyl oder 2-Ethylhexanoyl.

Es wurde weiterhin gefunden, daß man Phenylendiamine der Formel I 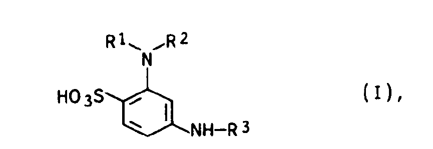
in der R¹ Wasserstoff, Formyl oder Oxalyl, R² Wasserstoff oder C₁-C₄-Alkyl und R³ Wasserstoff oder C₂-C₈-Alkanoyl bedeuten, mit der Maßgabe, daß R¹ und R² nicht gleichzeitig für Wasserstoff stehen, vorteilhaft erhält, wenn man 1,3-Diaminobenzol-4-sulfonsäure zunächst mit Ameisensäure oder Oxalsäure und gegebenenfalls anschließend mit einem Acylierungsmittel, das sich von einer C₂-C₈-Alkansäure ableitet, behandelt, dann gegebenenfalls in einem zweiten Schritt das resultierende Acylderivat der Formel II 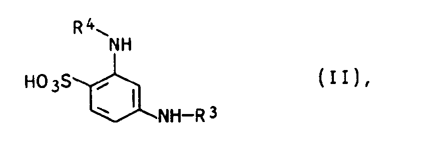
in der R⁴ Formyl oder Oxalyl bedeutet und R³ die obengenannte Bedeutung besitzt, mit einem Alkylierungsmittel der Formel III

R⁵-X (III),

in der R⁵ C₁-C₄-Alkyl und X eine Abgangsgruppe bedeuten, umsetzt und gegebenenfalls in einem dritten Schritt die Formyl- oder Oxalylgruppe und gegebenenfalls den C₂-C₈-Alkanoylrest hydrolytisch abspaltet.

Das erfindungsgemäße Verfahren wird zweckmäßig so durchgeführt, daß man 1,3-Diaminobenzol-4-sulfonsäure in wäßriger Lösung bei einer Temperatur von 90 bis 100°C mit Ameisensäure oder Oxalsäure behandelt, wobei die Säurekonzentration in der wäßrigen Lösung im allgemeinen 5 bis 98 Gew.%, vorzugsweise 5 bis 30 Gew.%, jeweils bezogen auf die Lösung, beträgt. Je Mol Diaminobenzol werden im allgemeinen 1,2 bis 4,0 Moläquivalente Säure verwendet. Die Verwendung von Ameisensäure ist bevorzugt.

Nach einer Reaktionsdauer, die üblicherweise 0,5 bis 5 Stunden, vorzugsweise 1,5 bis 3 Stunden beträgt, kann die resultierende 3-(N-Formylamino)-oder 3-(N-Oxalylamino)anilin-4-sulfonsäure bei einem pH-Wert von 1 bis 7, vorzugsweise 1 bis 3,5 oder 5 bis 7, gegebenenfalls unter Zugabe von Kochsalz, ausgefällt und abgesaugt werden. Es ist jedoch auch möglich, die anfallende Reaktionslösung direkt weiterzuverarbeiten.

Nach der Behandlung mit Ameisensäure oder Oxalsäure kann die Behandlung mit dem Acylierungsmittel, das sich von einer C₂-C₈-Alkansäure ableitet, erfolgen. Diese Behandlung wird üblicherweise in wäßrigem Reaktionsmedium vorgenommen.

Bei den genannten Acylierungsmitteln handelt es sich üblicherweise um die entsprechenden Carbonsäureanhydride, wobei auch gemischte Carbonsäureanhydride in Betracht kommen, oder um die entsprechenden Carbonsäurehalogenide. Beispielhaft seien Essigsäureanhydrid, Propionsäureanhydrid, Acetylchlorid oder Propionylchlorid genannt. Die Verwendung von Propionsäureanhydrid und insbesondere von Essigsäureanhydrid ist bevorzugt.

Je Mol Phenylendiamin werden üblicherweise 1,1 bis 2,0 Mol, vorzugsweise 1,1 bis 1,7 Mol, des Acylierungsmittels verwendet. Im allgemeinen läuft die Acylierung bei einer Temperatur von 10 bis 45°C , vorzugsweise 20 bis 30°C und bei einem pH-Wert von 3 bis 7,5, vorzugsweise 5,5 bis 6,5 ab.

Nach beendeter Umsetzung, die im allgemeinen 15 bis 90 Minuten, vorzugsweise 30 bis 60 Minuten, in Anspruch nimmt, kann das Acylierungsprodukt der Formel II (mit R³ = C₂-C₈-Alkanoyl) bei einem pH-Wert von 0,5 bis 7,5, vorzugsweise 3 bis 4,5 mittels Kochsalz ausgefällt werden. Es ist jedoch auch möglich, die anfallende Reaktionslösung direkt weiterzuverarbeiten.

In einer bevorzugten Verfahrensweise arbeitet man ohne das Acylierungsmittel, d.h. die Alkylierungsreaktion erfolgt direkt nach der Behandlung mit Ameisensäure oder Oxalsäure. Vorzugsweise arbeitet man dabei auch ohne Zwischenisolierung des Reaktionsproduktes. Bevor die Alkylierungsreaktion beginnt, stellt man den pH-Wert der wäßrigen Lösung auf 6 bis 7 ein.

Diejenigen Verbindungen der Formel I, in der R² für C₁-C₄-Alkyl steht, erhält man z.B. durch Umsetzung des Acylierungsprodukts II mit einem Alkylierungsmittel der Formel III

R⁵-X (III),

in der R⁵ für C₁-C₄-Alkyl und X für eine Abgangsgruppe steht.

Geeignete Abgangsgruppen x sind z.B. Halogen, wie Chlor, Brom oder Iod, oder ein Rest der Formel O-SO₂-C₆H₅, O-SO₂-C₆H₄-CH₃ oder O-SO₂-OR⁵, wobei R⁵ die obengenannte Bedeutung besitzt. Die Verwendung von C₁-C₄-Dialkylsulfaten, insbesondere von Dimethylsulfat oder Diethylsulfat ist bevorzugt.

Die Alkylierungsreaktion wird nach an sich bekannten Methoden in wäßrigem Reaktionsmedium in Gegenwart einer Base, z.B. Natrium- oder Kaliumhydroxid, durchgeführt, wobei die Verwendung von Natriumhydroxid bevorzugt ist. Je Mol Acylierungsprodukt II wendet man im allgemeinen 1,0 bis 2,0 Mol, vorzugsweise 1,2 bis 1,7 Mol, des Alkylierungsmittels III an. Je Moläquivalent des Alkylierungsmittels III werden in der Regel 1 bis 2 Mol, vorzugsweise 1,1 bis 1,3 Mol, an Base angewandt.

Die Reaktionstemperatur beträgt üblicherweise 10 bis 80°C. Beim Arbeiten mit Dimethylsulfat ist eine Temperatur von 10 bis 50°C, insbesondere von 20 bis 35°C bevorzugt, während beim Arbeiten mit Diethylsulfat der Temperaturbereich von 40 bis 80°C hervorzuheben ist.

Die Reihenfolge der Zugabe der Reagenzien ist beliebig. Die Umsetzung ist im allgemeinen nach einer Reaktionszeit von 5 bis 30 Minuten beendet und das Reaktionsprodukt kann dann nach an sich bekannten Methoden isoliert werden.

Man kann aber auch ohne Zwischenisolierung direkt die Formyl- oder Oxalylgruppe sowie den C₂-C₈-Alkanoylrest hydrolytisch abspalten. Dies kann entweder in saurem oder alkalischem wäßrigen Milieu geschehen.

Beim Arbeiten im sauren Milieu verwendet man beispielsweise Salzsäure oder Schwefelsäure als Agens und arbeitet bei einem pH-Wert von -0,5 bis +2, vorzugsweise -0,5 bis +0,5. Beim Arbeiten im alkalischen Milieu verwendet man z.B. 1 bis 6 Mol Natriumhydroxid oder Kaliumhydroxid, bezogen auf 1 Mol des zu entacylierenden Produkts, als Agens.

Sowohl im sauren als auch im alkalischen Milieu wird die hydrolytische Abspaltung unter Erwärmen auf Rückflußtemperatur vorgenommen. Nach einer Reaktionsdauer von 2 bis 3 Stunden ist die Abspaltung zu Ende, und man kann das Zielprodukt nach an sich bekannten Methoden isolieren.

Mittels des neuen Verfahrens, das sowohl in kontinuierlicher als auch in diskontinuierlicher Arbeitsweise vorgenommen werden kann, gelangt man in einfacher Weise zu den erfindungsgemäßen Phenylendiaminen der Formel I. Bei ihnen handelt es sich um wertvolle Zwischenprodukte für die Synthese von Farbstoffen, z.B. von Reaktivfarbstoffen, wie sie in der älteren Patentanmeldung EP-A-315 045 beschrieben sind.

Die folgenden Beispiele sollen die Erfindung näher erläutern.

### Beispiel 1

94 g 1,3-Diaminobenzol-4-sulfonsäure wurden in 430 ml Wasser und 70 g Ameisensäure drei Stunden unter Rückfluß gekocht. Nach Abkühlen auf 20°C wurde die entstandene 3-(N-Formylamino)anilin-4-sulfonsäure abgesaugt und in 500 g Wasser angerührt, wobei mit Natronlauge ein pH-Wert von 6,5 eingestellt wurde. Es wurden nun 70 g Essigsäureanhydrid hinzugegeben und dabei der pH-Wert durch Zugabe von Natronlauge zwischen 5,0 und 6,5 gehalten. Nach 30 Minuten wurde der pH-Wert mit Natronlauge auf 12,5 gestellt und 75 g Dimethylsulfat wurden eingetropft. Nach 30 Minuten wurde mit Salzsäure ein pH-Wert von -0,5 eingestellt und 3 Stunden unter Rückfluß gekocht. Nach dem Abkühlen wurde mit Natronlauge ein pH-Wert von 1,0 eingestellt, mit 150 g Natriumchlorid ausgesalzen und die ausgefallene 3-(N-Methylamino)anilin-4-sulfonsäure abgesaugt.

¹³C-NMR-Daten:
3-(N-Formylamino)anilin-4-sulfonsäure
δ (d⁶-DMSO): 173,6 (d), 153,3 (s), 148,0 (s), 131,6 (d), 120,6 (s), 106,4 (d), 105,5 (d)

3-(N-Methylamino)anilin-4-sulfonsäure
- δ (d⁶-DMSO):: 153,8 (s), 150,5 (s), 131,8 (d), 120,2 (s), 106,2 (d), 100,8 (d), 32,5 (q)

### Beispiel 2

115 g 3-(N-Acetylamino)anilin-4-sulfonsäure wurden in 430 ml Wasser und 70 g Ameisensäure 7 Stunden unter Rückfluß gekocht. Nach dem Abkühlen auf 20°C wurde anschließend mit Natronlauge ein pH von 12 eingestellt. Dann wurden 75 g Dimethylsulfat hinzugegeben und nach 40 Minuten wurde mit Salzsäure ein pH-Wert von -0,5 eingestellt. Nach 3 Stunden Erhitzen auf Rückflußtemperatur wurde mit 150 g Kochsalz ausgesalzen, mit Natronlauge ein pH-Wert von 1,0 eingestellt und die als Niederschlag ausgefallene 3-(N-Methylamino)anilin-4-sulfonsäure durch Filtration isoliert.

### Beispiel 3

94 g 1,3-Diaminobenzol-4-sulfonsäure wurden in 430 ml Wasser und 70 g Ameisensäure 3 Stunden unter Rückfluß gekocht. Bei 20°C wurden anschließend 100 ml Wasser hinzugegeben und der pH-Wert wurde mit Natronlauge auf 6,5 gestellt. Dann wurden 70 g Essigsäureanhydrid hinzugegeben und der pH-Wert mit Natronlauge dabei zwischen 5,5 und 6,5 gehalten. Anschließend wurde mit Natronlauge auf einen pH-Wert von 12,5 gestellt und mit 75 g Dimethylsulfat versetzt. Nach 30 Minuten wurden 110 g Natronlauge (50 gew.%ig) hinzugegeben und 3 Stunden bei 100°C unter Rückfluß gekocht. Anschließend wurden 150 ml Wasser abdestilliert und mit Salzsäure auf einen pH-Wert von 1 gestellt. Bei 20°C wurde die als Niederschlag ausgefallene 3-(N-Methylamino)anilin-4-sulfonsäure durch Filtration isoliert.

### Beispiel 4

47 g 1,3-Diaminobenzol-4-sulfonsäure wurden in 215 ml Wasser und 35 g Ameisensäure 3 Stunden unter Rückfluß gekocht. Der pH-Wert wurde dann mit Natronlauge auf 6,5 gestellt und bei 20°C wurden 35 g Essigsäureanhydrid zugetropft. Der pH-Wert wurde dabei mit Natronlauge zwischen 6,0 und 7,0 gehalten. Nach 1 Stunde wurde mit Salzsäure auf einen pH-Wert von 4,0 gestellt und 200 g Natriumchlorid hinzugegeben. Nach 5 Stunden Nachrühren wurde die als Niederschlag ausgefallene 1-(N-Acetylamino)-3-(N-formylamino)benzol-4-sulfonsäure durch Filtration isoliert.

¹³C-NMR-Daten:
1-(N-Acetylamino)-3-(N-formylamino)benzol-4-sulfonsäure
- δ (d⁶-DMSO):: 168,8 (s), 159,9 (d), 140,5 (s), 134,4 (s), 130,0 (s), 127,7 (d), 113,7 (d), 111,5 (d), 23,8 (q)

### Beispiel 5

220 g 3-(N-Formylamino)anilin-4-sulfonsäure wurden in 1500 ml Wasser bei einem pH-Wert von 5,0 bis 5,5 mit 140 g Essigsäureanhydrid umgesetzt. Bei einem pH-Wert von 7,0 wurde auf 50°C erwärmt und 200 g Diethylsulfat hinzugegeben. Mit Natronlauge wurde anschließend der pH-Wert auf 12,5 bis 13,0 gestellt und 2 Stunden nachgerührt. Anschließend wurde mit Salzsäure auf einen pH-Wert von 0 bis -0,5 gestellt und 3 Stunden bei 100°C unter Rückfluß gekocht. Nach dem Abkühlen wurde der pH-Wert mit Natronlauge auf 1,0 gestellt, 200 g Natriumchlorid hinzugegeben und die als Niederschlag ausgefallene 3-(N-Ethylamino)anilin-4-sulfonsäure abgesaugt.

¹³C-NMR-Daten:
3-(N-Ethylamino)anilin-4-sulfonsäure
- δ (d⁶-DMSO):: 142,7 (s), 137,7 (s), 128,6 (s und d), 109,1 (d), 104,8 (d), 34,4 (t), 13,5 (q)

### Beispiel 6

Man verfuhr analog Beispiel 4, verwendete jedoch anstelle von Essigsäureanhydrid 42 g Propionsäureanhydrid. Man erhielt 1-(N-Propionylamino)-3-(N-formylamino)benzol-4-sulfonsäure.

¹³C-NMR-Daten:
- δ (d⁶-DMSO):: 172,2 (s), 159,7 (d), 140,4 (s), 134,4 (s), 130,1 (s), 127,5 (d), 113,4 (d), 111,1 (d), 29,5 (t), 9,4 (q)

### Beispiel 7

Man verfuhr analog Beispiel 1, verwendete jedoch anstelle von Essigsäureanhydrid 86 g Propionsäureanhydrid. Man erhielt 3-(N-Methylamino)-anilin-4-sulfonsäure.

### Beispiel 8

97 g 1,3-Diaminobenzol-4-sulfonsäure wurden in 250 ml Wasser angerührt und nach Zusatz von 38 ml 98 gew.-%iger Ameisensäure 5 Stunden bei 95°C bis 100°C gekocht. Nach Abkühlen auf 30°C wurden 65 g Eis hinzugesetzt und mit 60,5 ml 50 gew.-%iger Natronlauge ein pH-Wert von 6,5 bis 7,0 eingestellt. Anschließend wurden 45 ml 50 gew.-%ige Natronlauge und 70 ml Dimethylsulfat hinzugegegeben. Nach einer Nachrührphase von 30 Minuten wurde mit 43 ml 96 gew.-%iger Schwefelsäure angesäuert und 3 Stunden bei 100°C gerührt. Nach dem Abkühlen wurde die als Niederschlag ausgefallene 3-(N-Methylamino)anilin-4-sulfonsäure durch Filtration isoliert.

## Patentansprüche

1. Phenylendiamine der Formel I 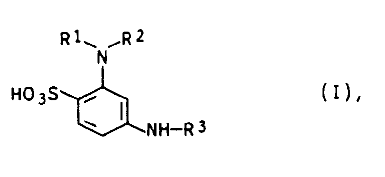 in der
R¹ Wasserstoff, Formyl oder Oxalyl,
R² Wasserstoff oder C₁-C₄-Alkyl und
R³ Wasserstoff oder C₂-C₈-Alkanoyl bedeuten,
mit der Maßgabe, daß R¹ und R² nicht gleichzeitig für Wasserstoff stehen und daß, wenn R² Methyl oder Ethyl bedeutet, R¹ und R³ nicht gleichzeitig für Wasserstoff stehen.

2. Verfahren zur Herstellung von Phenylendiaminen der Formel I 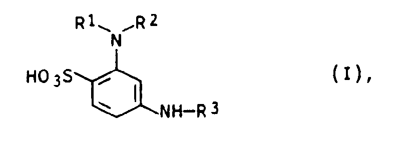 in der
R¹ Wasserstoff, Formyl oder Oxalyl,
R² Wasserstoff oder C₁-C₄-Alkyl und
R³ Wasserstoff oder C₂-C₈-Alkanoyl bedeuten,
mit der Maßgabe, daß R¹ und R² nicht gleichzeitig für Wasserstoff stehen, dadurch gekennzeichnet, daß man 1,3-Diaminobenzol-4-sulfonsäure zunächst mit Ameisensäure oder Oxalsäure und gegebenenfalls anschließend mit einem Acylierungsmittel, das sich von einer C₂-C₈-Alkansäure ableitet, behandelt, dann gegebenenfalls in einem zweiten Schritt das resultierende Acylderivat der Formel II 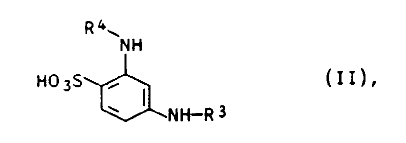 in der R⁴ Formyl oder Oxalyl bedeutet und R³ die obengenannte Bedeutung besitzt, mit einem Alkylierungsmittel der Formel III
R⁵-X (III),
in der R⁵ C₁-C₄-Alkyl und X eine Abgangsgruppe bedeuten, umsetzt und gegebenenfalls in einem dritten Schritt die Formyl- oder Oxalylgruppe und gegebenenfalls den C₂-C₈-Alkanolylrest hydrolytisch abspaltet.

## Claims

1. Phenylenediamines of the formula I 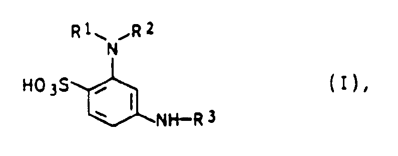 where
R¹ is hydrogen, formyl or oxalyl,
R² is hydrogen or C₁-C₄-alkyl, and
R³ is hydrogen or C₂-C₈-alkanoyl,
with the proviso that R¹ and R² are not both hydrogen and that when R² is methyl or ethyl, R¹ and R³ are not both hydrogen.

2. A process for preparing phenylenediamines of the formula I 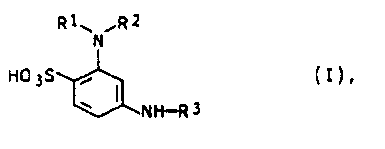 where
R¹ is hydrogen, formyl or oxalyl,
R² is hydrogen or C₁-C₄-alkyl, and
R³ is hydrogen or C₂-C₈-alkanoyl,
with the proviso that R¹ and R² are not both hydrogen, which comprises treating 1,3-diaminobenzene-4-sulphonic acid first with formic acid or oxalic acid and optionally subsequently with an acylating agent derived from a C₂-C₈-alkanoic acid, then optionally in a second step reacting the resulting acyl derivative of the formula II 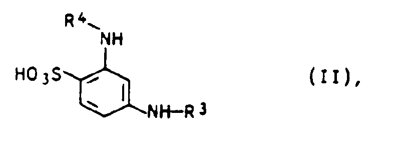 where R⁴ is formyl or oxalyl and R³ is as defined above, with an alkylating agent of the formula III
R⁵-X (III),
where R⁵ is C₁-C₄-alkyl and X is a leaving group, and optionally in a third step eliminating the formyl or oxalyl group and optionally the C₂-C₈-alkanol radical by hydrolysis.

## Revendications

1. Phénylènediamines de formule I 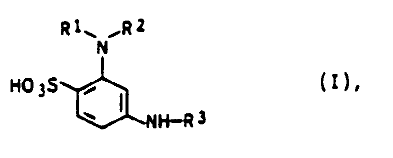 dans laquelle
R¹ représente un atome d'hydrogène ou un reste formyle ou oxalyle,
R² représente un atome d'hydrogène ou un reste alkyle en C₁-C₄ et
R³ représente un atome d'hydrogène ou un reste alcanoyle en C₂-C₈,
étant spécifié que R¹ et R² ne sont pas mis en même temps pour des atomes d'hydrogène et que, quand R² représente un reste méthyle ou éthyle, R¹ et R³ ne sont pas mis en même temps pour des atomes d'hydrogène.

2. Procédé de préparation de phénylènediamines de formule I 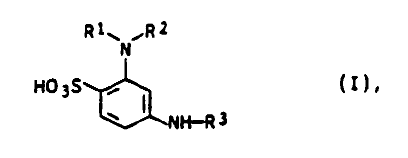 dans laquelle
R¹ représente un atome d'hydrogène ou un reste formyle ou oxalyle,
R² représente un atome d'hydrogène ou un reste alkyle en C₁-C₄ et
R³ représente un atome d'hydrogène ou un reste alcanoyle en C₂-C₈,
étant spécifié que R¹ et R² ne sont pas mis en même temps pour des atomes d'hydrogène, caractérisé en ce qu'on traite de l'acide 1,3-diaminobenzène-4-sulfonique, tout d'abord par de l'acide formique ou de l'acide oxalique, puis éventuellement par un agent d'acylation qui dérive d'un acide alcanoïque en C₂-C₈, on fait éventuellement réagir ensuite, dans une deuxième étape, le dérivé acylé résultant de formule II 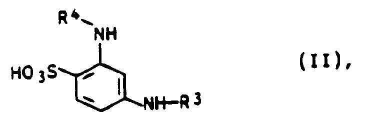 dans laquelle R⁴ représente un reste formyle ou oxalyle et R³ a la signification susmentionnée, avec un agent d'alkylation de formule III
R⁵-X (III)
dans laquelle R⁵ represente un reste alkyle en C₁-C₄ et X est un groupement labile, et on élimine éventuellement par hydrolyle, dans une troisième étape, le groupement formyle ou oxalyle et éventuellement le reste alcanoyle en C₂-C₈.
